Europäisches Patentamt

European Patent Office

Office européen des brevets

(19)

(11) Veröffentlichungsnummer : **0 336 085 B1**

(12) **EUROPÄISCHE PATENTSCHRIFT**

(45) Veröffentlichungstag der Patentschrift :
08.07.92 Patentblatt 92/28

(51) Int. Cl.⁵ : **A61B 17/22**

(21) Anmeldenummer : 89102831.8

(22) Anmeldetag : 18.02.89

(54) **Target.**

(30) Priorität : 05.03.88 DE 3807304

(43) Veröffentlichungstag der Anmeldung :
11.10.89 Patentblatt 89/41

(45) Bekanntmachung des Hinweises auf die
Patenterteilung :
08.07.92 Patentblatt 92/28

(84) Benannte Vertragsstaaten :
CH ES FR IT LI NL

(56) Entgegenhaltungen :
EP-A- 0 030 355
EP-A- 0 211 680
US-A- 4 373 532

(73) Patentinhaber : **DORNIER MEDIZINTECHNIK
GMBH
Postfach 1128
W-8034 Germering 1 (DE)**

(72) Erfinder : **Denk, Roland, Dipl.-Phys.
Rankestrasse 11
W-8000 München 40 (DE)**
Erfinder : **Kreibich, Rainer
Ysenburgstrasse 12
W-8000 München 2 (DE)**
Erfinder : **Viebach, Thomas, Dipl.-Ing.
Ammenhof 2
W-8021 Pähl (DE)**

(74) Vertreter : **Landsmann, Ralf, Dipl.-Ing.
DORNIER GMBH - Patentabteilung - Kleeweg
3
W-7990 Friedrichshafen 1 (DE)**

## Beschreibung

Die Erfindung betrifft ein Target zur Bestimmung der Zertrümmerungswirkung von Geräten zur extrakorporalen Stosswellenbehandlung, insbesondere zur Steinzerkleinerung nach dem Oberbegriff des Anspruchs 1.

Bisher wurden die Zertrümmerungswirkungen solcher Geräte an Kunststeinen bestimmt, das heisst ein Stein aus Gips oder einem ähnlichen Material wurde in den Fokus des Gerätes gebracht und dann mit Stosswellen behandelt. Gemessen wurden dann zum Beispiel die Kratertiefe und/oder der Volumendefekt. Diese Messwerte wurden dann in Relation zur Zertrümmerung realer Gallen- oder Nierensteine gebracht. Dieses Verfahren ist, ebenso wie die Druckmessung im therapeutischen Fokus, unbefriedigend, da das Zertrümmerungsverhalten des Steins nicht ausreichend simuliert wird.

Aufgabe der Erfindung ist es daher, ein Target vorzuschlagen, mit dem die Zertrümmerungswirkung von Geräten zur extrakorporalen Stosswellenbehandlung einfach und genau bestimmt werden kann.

Diese Aufgabe wird erfindungsgemäss gelöst von einem Target mit den Merkmalen der Patentansprüche. Behälter zur Aufnahme des Targets sind Gegenstände von Unteransprüchen.

Die Erfindung beruht auf der Idee, die zu zerstörenden Konkremente möglichst exakt nachzubilden und durch den sack- oder siebartigen Träger mit definierter Maschen- oder Lochweite eine automatische Erkennung der Zertrümmerungswirkung zu ermöglichen. Der erfindungsgemässe sack- oder siebartige Träger wird in den therapeutischen Fokus positioniert. Die Maschenweite des Trägers entspricht der maximal zulässigen Grösse a der Steinfragmente. Das Abbruchkriterium des Zertrümmerungsvorgangs ist, dass der Träger leer ist, das heisst, dass alle Steinfragmente kleiner a sind. Auf diese Weise ist eine leicht nachprüfbare objektive Messung der Zertrümmerungswirkung möglich.

Die Anzahl der notwendigen "Schüsse" und deren jeweiliger Energieinhalt, die zur Leerung des Trägers notwendig sind, sind ein objektives Maß für die Zertrümmerungswirkung des jeweiligen Gerätes. Einflüsse von Inhomogenitäten im Stosswellenkegel (durch das Gewebe) Amplitudenform der Druckwelle und Ortungsgenauigkeit können so einfach bestimmt werden. Härte, Wassergehalt, chemische Zusammensetzung und Strukturierung der Steinproben können im weiten Bereich variiert werden.

Bevorzugt besteht der Träger aus Kunststoff, Gummi oder einem stosswellenbeständigen Metall. Er ist, als einfachste Lösung in einem wassergefüllten Behälter eingebracht. Die Wasserfüllung simuliert das Koppelmedium und das Körpergewebe.

Für die Kugeln oder Körner wird bevorzugt Gips, Zement, Keramik oder ein Kunststoff verwendet. Die Kugeln können auch Mikrospheres enthalten.

Eine bevorzugte Möglichkeit ist, den Behälter mit Kugeln gleichen Durchmessers zu füllen. Die Kugeln können homogen und isotrop sein, wodurch definierte Zertrümmerungsbedingungen geschaffen werden. Eine Standardisierung der Messungen kann dadurch erfolgen, dass Kugeln mit definierten Durchmessern, im Bereich von ca. 3 bis 30 mm, verwendet werden. Die Maschenweite a liegt bevorzugt bei 1 bis 3 mm, was der Bruchstückgrösse zum spontanen Abgang in der Niere entspricht.

Möglich ist auch, den Träger mit einer Mehrzahl von Kugeln oder Körnern zu füllen, die unterschiedliche Durchmesser haben. Dies ist realistisch für die Behandlung von Patienten mit einer Mehrzahl von Steinen. Eine Freiheit besteht auch in der Anzahl der Körner oder Kugeln. So kann bereits ein Konkrement zu einer Darstellung einer Behandlung ausreichen.

Die Kugeln oder Körner können auch inhomogen aufgebaut sein, z.B. eine Schalenstruktur besitzen. Das heisst, ihre elastischen Eigenschaften, wie Härte oder Sprödigkeit, variieren mit dem Radius. So kann ein relativ weicher Kern von einer härteren Schale gegeben sein, wie dies bei realen Konkrementen auch häufig vorkommt.

Das Target kann bei Geräten mit einer Wanne innerhalb der Wanne mit einem einfachen Halter in den Fokus gehängt werden. Ist keine Wanne vorhanden, sondern nur eine Ankoppelfläche, so wird das Target bevorzugt in einen Behälter gebracht, der mit Koppelflüssigkeit gefüllt ist, die das Gewebe des Patienten imitiert und der mindestens eine Koppelfläche aufweist, durch die die Stosswellen ohne besondere Verluste einleitbar sind oder durch die die Einkoppelfolien realer Geräte simuliert werden. Dieser Behälter dient gleichzeitig zum Aufsammeln der Bruchstücke. Möglich ist, den Behälter so auf die Stosswellenquelle zu setzen, dass die Bruchstücke aus dem Stosswelleneindringbereich herausfallen, zum Beispiel dadurch, dass die Koppelfläche leicht geneigt angeordnet ist. Bei jedem Schuß durch die Koppelfläche fallen dann die darauf liegenden Konkremente durch die leichte Druckbewegung zur Seite und damit aus dem Wirkungsbereich der Stosswelle.

Möglich ist auch, eine Folie zum Auffangen und/oder Entfernen der Konkremente innerhalb des Behälters vorzusehen. Kunststoffolien, insbesondere wenn sie dünner als ca. 50 μm sind, stören den Stosswellendurchgang nicht. Die Folie kann bevorzugt geneigt angeordnet sein und sammelt dann automatisch die Bruchstücke in einem Eck.

Die Erfindung wird anhand einer Figur näher erläutert.

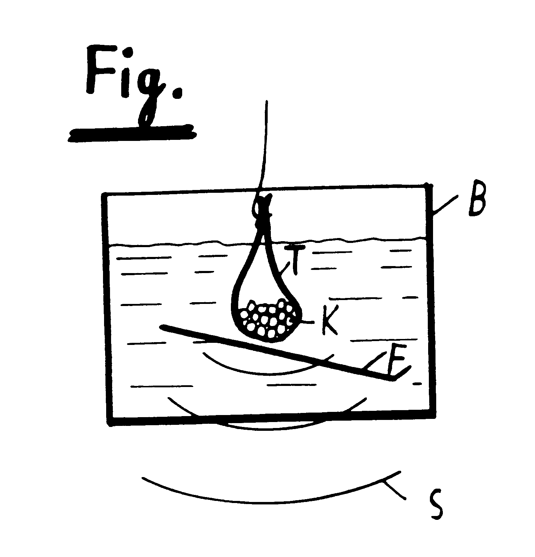

Die Figur zeigt den Behälter B, der teilweise mit einer Koppelflüssigkeit, z.B. Wasser, gefüllt ist. Der Behälter B weist an seiner Unterseite eine Koppelfläche auf, die für die hier schematisch gezeigten, fokussierten Stosswellen S durchlässig ist. Im Behälter B befindet sich der Träger T, der mit den Kugeln K gefüllt ist. Der Träger T ist im Fokuspunkt der konvergierenden Stosswellenfronten angeordnet. Weiter ist die Folie F vorgesehen, die die Bruchstücke der Kugeln K, die durch die Maschen des Trägers T fallen, auffängt. Aufgrund ihrer geneigten Anordnung sammeln sich die Bruchstücke im rechten Bereich der Folie F, z.B. in der dort angedeuteten Rinne.

## Patentansprüche

1. Target zur Bestimmung der Zertrümmerungswirkung von Geräten zur extrakorporalen Stosswellenbehandlung, insbesondere zur Steinzerkleinerung, mit einer Haltevorrichtung zum Positionieren in den Wirkbereich oder Fokus, **gekennzeichnet** durch einen sack- oder siebartigen Träger (T) mit Durchlässen, Maschen oder Bohrungen mit definierter Maschenweite a oder definiertem Durchmesser a und ein oder mehrere Körner oder Kugeln (K) mit einer Korngrösse (Durchmesser) grösser a.

2. Target nach Anspruch 1, dadurch **gekennzeichnet**, dass der Träger (T) aus Gummi, Kunststoff oder Metall besteht.

3. Target nach einem der vorhergehenden Ansprüche, dadurch **gekennzeichnet,** dass die Körner oder Kugeln (K) aus Gips, Zement, Keramik oder Kunststoff (z.B. mit Mikrospheres) bestehen.

4. Target nach einem der vorhergehenden Ansprüche, dadurch **gekennzeichnet,** dass die Kugeln (K) homogen und/oder isotrop sind.

5. Target nach einem der vorhergehenden Ansprüche, dadurch **gekennzeichnet,** dass alle Körner oder Kugeln (K) den gleichen Durchmesser haben, der im Bereich von ca. 3 bis 30 mm liegen kann.

6. Target nach einem der Ansprüche 1 bis 3, dadurch **gekennzeichnet**, dass die Körner oder Kugeln inhomogen sind, wobei sich deren elastische Eigenschaften und/oder chemische Zusammensetzung, bevorzugt in Abhängigkeit vom Radius, ändern (Schalenstruktur).

7. Target nach einem der Ansprüche 1 bis 4, dadurch **gekennzeichnet,** dass die Körner oder Kugeln (K) als Gemisch mit unterschiedlichen Korngrössen oder Durchmessern vorliegen.

8. Behälter (B) mit einem Target nach einem der Ansprüche 1-7, dadurch **gekennzeichnet,** dass er mit einer Koppelflüssigkeit gefüllt ist und mindestens eine Koppelfläche aufweist, durch die Stosswellen (S) ohne wesentliche Verluste einleitbar sind.

9. Behälter (B) nach Anspruch 8, **gekennzeichnet** durch eine Folie (F) zum Auffangen und/oder Entfernen der Bruchstücke.

## Claims

1. Target for determining the disintegration effect of apparatus for extra-corporeal shock wave treatment, in particular for disintegrating stones, with a holder device for positioning in the operating range or focus, **characterized** by a sack or sieve-like carrier (T) having apertures, meshes or bores with a specific mesh width a or specific diameter a and one or more grains or spheres (K) having a grain size (diameter) which is greater than a.

2. Target according to Claim 1, **characterized** in that the carrier (T) is made from rubber, plastics material or metal.

3. Target according to either of the preceding claims, **characterized** in that the grains or spheres (K) are made from plaster, cement, ceramics or plastics material (e.g. with microspheres).

4. Target according to any one of the preceding claims, **characterized** in that the spheres (K) are homogeneous and/or isotropic.

5. Target according to any one of the preceding claims, **characterized** in that all the grains or spheres (K) have the same diameter which can be in the range from approximately 3 to 30 mm.

6. Target according to any one of Claims 1 to 3, **characterized** in that the grains or spheres are irregular, their resilient properties and/or chemical composition varying, preferably as a function of the radius (shell structure).

7. Target according to any one of Claims 1 to 4, **characterized** in that the grains or spheres (K) are present as a mixture with different grain sizes or diameters.

8. Container (B) having a target according to any one of Claims 1 to 7, **characterized** in that it is filled with a coupling fluid and comprises at least one coupling face through which the shock waves (S) can be conducted without substantial losses.

9. Container (B) according to Claim 8, **characterized** by a foil (F) for collecting and/or removing the fragments.


## Revendications

1. Cible pour la détermination de l'effet de fragmentation d'appareils pour le traitement extracorporel par ondes de choc, en particulier pour la fragmentation de calculs, comprenant un dispositif de retenue pour le positionnement dans la zone d'action ou le foyer, **caractérisée en ce** qu'elle comprend un support (T) en forme de poche ou de crible avec des passages, mailles ou trous avec une ouverture de maille a définie ou un diamètre a défini, et un ou plusieurs grains ou billes (K) d'une grosseur de grains (diamètre) plus grande que a.

2. Cible selon la revendication 1, **caractérisée en ce** que le support (T) est réalisé en caoutchouc, matière plastique ou métal.

3. Cible selon l'une des revendications précédentes, **caractérisée en ce** que les grains ou billes (K) sont constitués de plâtre de ciment, de céramique ou de matière plastique (par exemple avec des microsphères).

4. Cible selon l'une des revendications précédentes, **caractérisée en ce** que les billes (K) sont homogènes et/ou isotropes.

5. Cible selon l'une des revendications précédentes, **caractérisée en ce** que tous les grains ou billes (K) ont le même diamètre qui peut se situer à l'intérieur de la plage d'environ 3 à 30 mm.

6. Cible selon l'une des revendications 1 à 3, **caractérisée en ce** que les grains ou billes ne sont pas homogènes, leurs propriétés élastiques et/ou leur composition chimique variant de préférence en fonction du rayon (structure de peau).

7. Cible selon l'une des revendications 1 à 4, **caractérisée en ce** que les grains ou billes (K) se présentent sous la forme d'un mélange avec des grosseurs de grains ou des diamètres variables.

8. Récipient (B) avec une cible selon l'une des revendications 1 à 7, **caractérisé en ce** qu'il est rempli d'un liquide de couplage et présente au moins une surface de couplage par laquelle les ondes de choc (S) peuvent être transmises sans pertes notables.

9. Récipient (B) selon la revendication 8, **caractérisé en ce** qu'il contient une feuille (F) pour l'interception et/on l'élimination des fragments.

Fig.